# EUROPEAN PATENT SPECIFICATION

(11) **EP 3 197 493 B1**
(45) Date of publication and mention of the grant of the patent: **10.03.2021**
(21) Application number: 15777810.1
(22) Date of filing: 25.09.2015
(51) Int. Cl.: A61K 39/395, C07K 16/22, A61P 35/00, A61K 31/282

(54) **METHODS OF REVERSING CACHEXIA AND PROLONGING SURVIVAL COMPRISING ADMINISTERING A GDF15 MODULATOR AND AN ANTI-CANCER AGENT**
VERFAHREN ZUR UMKEHRUNG VON KACHEXIE UND VERLÄNGERUNG DER ÜBERLEBENSDAUER MIT DER VERABREICHUNG EINES GDF15-MODULATORS UND EINES ANTIKREBSMITTELS
PROCÉDÉS D'INVERSION DE LA CACHEXIE ET DE PROLONGATION DE LA SURVIE, COMPRENANT L'ADMINISTRATION D'UN MODULATEUR DE GDF15 ET D'UN AGENT ANTICANCÉREUX

(30) Priority: 25.09.2014 US 201462055203 P
(43) Date of publication of application: 02.08.2017
(73) Proprietor: Aveo Pharmaceuticals Inc., Cambridge, MA 02142 (US)
(72) Inventor: GYURIS, Jeno, Lincoln, MA 01773 (US); LERNER, Lorena, Newton Centre, MA 02459 (US); LIN, Jie, West Roxbury, MA 02132 (US)
(74) Representative: Pohlman, Sandra M.
(86) International application number: PCT/US2015/052247
(87) International publication number: WO 2016/049470

(56) References cited:
- WO-A1-2005/099746
- WO-A1-2014/100689
- JOHNEN HEIKO ET AL: "Tumor-induced anorexia and weight loss are mediated by the TGF-beta superfamily cytokine MIC-1", NATURE MEDICINE, NATURE PUBLISHING GROUP, NEW YORK, NY, US, vol. 13, no. 11, 1 November 2007 (2007-11-01), pages 1333-1340, XP002607045, ISSN: 1078-8956, DOI: 10.1038/NM1677 [retrieved on 2007-11-04]
- JEFFREY S DAMRAUER ET AL: "Chemotherapy-induced muscle wasting: association with NF-kappaB cancer cachexia", BASIC APPLIED MYOLOGY, UNIPRESS, IT, vol. 18, no. 5, 1 January 2008 (2008-01-01), pages 139-148, XP002733150, ISSN: 1120-9992
- VICKY W. W. TSAI ET AL: "Anorexia/cachexia of chronic diseases: a role for the TGF-&bgr; family cytokine MIC-1/GDF15", JOURNAL OF CACHEXIA, SARCOPENIA AND MUSCLE, vol. 3, no. 4, 31 December 2012 (2012-12-31), pages 239-243, XP055105895, ISSN: 2190-5991, DOI: 10.1007/s13539-012-0082-6
- THOMAS ET AL: "Loss of skeletal muscle mass in aging: Examining the relationship of starvation, sarcopenia and cachexia", CLINICAL NUTRITION, CHURCHILL LIVINGSTONE, LONDON, GB, vol. 26, no. 4, 26 July 2007 (2007-07-26), pages 389-399, XP022159281, ISSN: 0261-5614, DOI: 10.1016/J.CLNU.2007.03.008

## Description

### BACKGROUND OF THE INVENTION

A large majority of advanced cancer patients experience progressive weight loss associated with anorexia, malnutrition, anemia, inflammation and suppression of immune functions. Collectively, this series of complex and inter-related symptoms have been described as Cancer Anorexia-Cachexia Syndrome (CACS). CACS is associated with muscle and fat mass loss, decreased quality of life, reduced response to anti-cancer therapies, increased treatment toxicity and reduced survival. (See, e.g., Thomas et al., 2007, CLINICAL NUTRITION, 26:389-399; Tsai et αl., 2012, J CACHEXIA SARCOPENIA MUSCLE, 3:239-243). Further, certain chemotherapeutic treatments used to treat various cancers have been shown to induce or contribute to cachexia. (See, e.g., Damrauer et al., 2008, BASIC APPLIED MYOLOGY, 18(5):139-148). In particular, subjects treated with platinum-based therapies, such as carboplatin and oxaliplatin, may experience dose-limiting, harmful, and sometimes fatal cachexia.

Growth Differentiation Factor-15 (GDF15) is a member of the transforming growth factor-beta (TGF-β) superfamily of proteins, which comprise a large group of multifunctional proteins that serve as regulators of cell proliferation and differentiation. Prominent members of this family include the TGF-βs 1-5, activins, bone morphogenetic proteins (BMPs) that serve as regulators of bone, cartilage and other tissue types, and other proteins involved in cellular regulation, such as glial cell-line derived neurotrophic factor (GDNF), and myostatin (also known as GDF-8). GDF15 was isolated early on from such tissues as prostate and placenta, and has been known by the additional names macrophage inhibitory cytokine 1 (or MIC1), NSAID-activated gene 1 protein (or NAG1), NSAID-regulated gene 1 protein (or NRG-1), placental TGF-beta (or PTGFB), placental bone morphogenetic protein (or PLAB), and prostate differentiation factor (or PDF).

Monoclonal antibodies against GDF15 have been recognized as potential anti-cachexia therapeutic agents. See, *e.g.,* U.S. Patent No. 8,192,735 and WO 2014/100689.

### SUMMARY OF THE INVENTION

The present inventors have discovered that, among other things, inhibition of GDF15 can reverse chemotherapy-induced cachexia. Accordingly, inhibition of GDF15 allows enhanced treatment by reducing the dose-limiting cachexia caused by such chemotherapeutics. The present inventors have also discovered that prevention and/or reversal of cachexia by administration of a GDF15 modulator is useful for the increase of overall survival in subjects treated with anti-cancer agents. Additionally, the inventors have discovered that treatment of cachexia can be integrated into anti-cancer treatment regimens to increase the therapeutic benefit .

In certain embodiments, the present invention relates to at least one chemotherapeutic anti-cancer agent and at least one anti-GDF15 antibody or GDF15-binding fragment thereof for use in increasing the overall survival in a subject having chemotherapy-induced cachexia.

In certain embodiments, administration of the anti-GDF15 antibody or GDF15-binding fragment thereof and the chemotherapeutic anti-cancer agent prolongs mean survival in a first patient population with chemotherapy-induced cachexia relative to a second patient population with chemotherapy-induced cachexia who do not receive the GDF15 modulator.

In certain embodiments the anti-cancer agent is selected from the group consisting of: capecitabine, gemcitabine, doxorubicin, cisplatin, carboplatin and oxaliplatin. In certain embodiments, the GDF15 modulator is an anti-GDF15 antibody, or a GDF15-binding fragment thereof.

### BRIEF DESCRIPTION OF THE FIGURES

FIG. **1** shows a graph illustrating the effects of treatment with GDF15 inhibitory antibody on survival of mice with tumors.
**FIG. 2** shows a graph illustrating the effects of combined treatment with cisplatin and a GDF15 inhibitory antibody on chemotherapy-induced cachexia in mice.
**FIG. 3** shows a graph illustrating the effects of combined treatment with carboplatin and a GDF15 inhibitory antibody on chemotherapy-induced cachexia in mice.
**FIG. 4** shows a graph illustrating the effects of combined treatment with oxaliplatin and a GDF15 inhibitory antibody on chemo-induced cachexia in mice.

### DETAILED DESCRIPTION

### I. GDF15 Modulators

As used herein a "GDF15 modulator" is understood to mean an agent that reduces or inhibits GDF15 activity or the activity of the GDF15 pathway, which can result from reduced expression, amount, or biological activity or function, of GDF15 or the GDF15 pathway. GDF15 modulators or modulating agents useful in the practice of the invention may comprise an anti-GDF15 antibody, an anti-GDF15 receptor antibody, soluble GDF15 mimetics or analogs that prevent GDF15 from binding to its cognate binding partner, a soluble GDF15 receptor mimetic or analog that prevents GDF15 from binding to its cognate binding partner. Additional exemplary GDF15 modulating agents include small molecule inhibitors of GDF15 or a GDF15 receptor, interfering nucleic acids (for example, interfering RNA or antisense nucleic acids, such as antisense DNA or RNA) that interfere with expression of endogenous GDF15 or a cognate receptor.

Antibodies against GDF15, GDF15-binding fragments thereof, and methods for their use have been described in U.S. Patent No. 8,192,735; WO 2014/100689 (corresponding to U.S. Patent Publication No. US 2014-0193427-A1); and International Patent Application Nos. PCT/US2015/036790 and PCT/US2015/036794.

The use of an anti GDF15 antibody to reverse weight loss in tumor bearing mice has been described in Heiko et al., 2007, NATURE MEDICINE, 13(11):1333-1340. A GDF15 (MIC-1) inhibitory agent for use in modulating the appetite and/or body weight loss has been described in WO 2005/099746.

In a preferred embodiment, the GDF15 modulating agent can comprise an anti-GDF15 antibody, which is humanized or human. As used herein, unless otherwise indicated, the term "antibody" is understood to mean an intact antibody (*e.g*., an intact monoclonal antibody) or antigen-binding fragment of an antibody, including an intact antibody or antigen-binding fragment of an antibody (*e.g*., a phage display antibody including a fully human antibody, a semisynthetic antibody or a fully synthetic antibody) that has been optimized, engineered or chemically conjugated. Examples of antibodies that have been optimized are affinity-matured antibodies. Examples of antibodies that have been engineered are Fc optimized antibodies, and multispecific antibodies (*e.g*., bispecific antibodies). Examples of antigen-binding fragments include Fab, Fab', F(ab')₂, Fv, single chain antibodies (*e.g*., scFv), minibodies and diabodies. An antibody conjugated to a toxin moiety is an example of a chemically conjugated antibody.

In certain embodiments, the antibody comprises: (a) an immunoglobulin heavy chain variable region comprising the structure CDR_{H1}-CDR_{H2}-CDR_{H3} and (b) an immunoglobulin light chain variable region, wherein the heavy chain variable region and the light chain variable region together define a single binding site for binding GDF15 or a GDF15 receptor. The CDR_{H1}, CDR_{H2}, and CDR_{H3} sequences are interposed between immunoglobulin framework (FR) sequences. In certain other embodiments, the antibody comprises (a) an immunoglobulin light chain variable region comprising the structure CDR_{L1}-CDR_{L2}-CDR_{L3}, and (b) an immunoglobulin heavy chain variable region, wherein the IgG light chain variable region and the IgG heavy chain variable region together define a single binding site for binding GDF15 or a GDF15 receptor. The CDR_{L1}, CDR_{L2}, and CDR_{L3} sequences are interposed between immunoglobulin FR sequences. In certain other embodiments, the antibody comprises: (a) an immunoglobulin heavy chain variable region comprising the structure CDR_{H1}-CDR_{H2}-CDR_{H3} and (b) an immunoglobulin light chain variable region comprising the structure CDR_{L1}-CDR_{L2}-CDR_{L3}, wherein the heavy chain variable region and the light chain variable region together define a single binding site for binding GDF15 or a GDF15 receptor. Exemplary anti-GDF15 antibodies are described, for example, in U.S. Patent Publication No. US 2014-0193427-A1.

Exemplary anti-GDF15 antibodies useful in the methods and compositions of the invention may, for example, include a heavy chain variable region comprising any one of the nine sets of CDR_{H1}, CDR_{H2}, and CDR_{H3} region sequences set forth in Table 1 below.

**TABLE 1**

| | CDR_{H1} | CDR_{H2} | CDR_{H3} |
|---|---|---|---|
| 1 | DYNMD (SEQ ID NO:1) | QINPNNGGIFFNQKFKG (SEQ ID NO:4) | EAITTVGAMDY (SEQ ID NO:13) |
| 2 | DYNMD (SEQ ID NO:1) | QINPNNGGIFFNQKFQG (SEQ ID NO:5) | EAITTVGAMDY (SEQ ID NO:13) |
| 3 | DYNMD (SEQ ID NO:1) | QINPYNHLIFFNQKFQG (SEQ ID NO:6) | EAITTVGAMDY (SEQ ID NO:13) |
| 4 | DYNMD (SEQ ID NO:1) | QINPNNGLIFFNQKFQG (SEQ ID NO:7) | EAITTVGAMDY (SEQ ID NO:13) |
| 5 | DYNMD (SEQ ID NO:1) | QINPNNGLIFFNQKFKG (SEQ ID NO:8) | EAITTVGAMDY (SEQ ID NO:13) |
| 6 | DYNMD (SEQ ID NO:1) | QINPYNHLIFFNQKFKG (SEQ ID NO:9) | EAITTVGAMDY (SEQ ID NO:13) |
| 7 | TYGMGVS (SEQ ID NO:2) | HIYWDDDKRYNPSLKS (SEQ ID NO:10 | RGYDDYWGY (SEQ ID NO:14) |
| 8 | TYGMGVS (SEQ ID NO:2) | HIYWDDDKRYNPSLKT (SEQ ID NO:11) | RGYDDYWGY (SEQ ID NO:14) |
| 9 | TYGMGVG (SEQ ID NO:3) | DIW-WDDDKYYNPSLKS (SEQ ID NO:12) | RGHYSAMDY (SEQ ID NO:15) |

Exemplary anti-GDF15 antibodies useful in the methods and compositions of the invention may, for example, include a light chain variable region comprising any one of the four sets of CDR_{L1}, CDR_{L2}, and CDR_{L3} region sequences set forth in Table 2 below.

**TABLE 2**

| | CDRL₁ | CDRL₂ | CDRL₃ |
|---|---|---|---|
| 1 | RTSENLHNYLA (SEQ ID NO:16) | DAKTLAD (SEQ ID NO:18) | QHFWSSPYT (SEQ ID NO:21) |
| 2 | RTSENLHNYLA (SEQ ID NO:16) | DAKTLAD (SEQ ID NO:18) | QHFWSDPYT (SEQ ID NO:22) |
| 3 | KASQNVGTNVA (SEQ ID NO:17) | SASYRYS (SEQ ID NO:19) | QQYNNYPLT (SEQ ID NO:23) |
| 4 | KASQNVGTNVA (SEQ ID NO:17) | SPSYRYS (SEQ ID NO:20) | QQYNSYPHT (SEQ ID NO:24) |

Exemplary anti-GDF15 antibodies useful in the practice of the invention are described in U.S. Patent Publication No. US 2014-0193427-A1, including 01G06, 03G05, 04F08, 06C11, 08G01, 14F11, 17B11, as well as human or humanized forms thereof. In certain embodiments, the antibodies disclosed herein (*e.g*., 01G06, 03G05, 04F08, 06C11, 08G01, 14F11, or 17B11, or humanized forms thereof) are used to increase the overall survival in a subject having chemotherapy-induced cachexia, wherein administration of the antibody and chemotherapeutic anti-cancer agent prolongs mean survival in a first patient population with cancer anorexia-cachexia syndrome relative to a second patient population with cancer anorexia-cachexia syndrome who do not receive the GDF15 modulator.

In a preferred embodiment, an anti-GDF15 antibody useful in the practice of the invention is referred to as 01G06 in US 2014/0193427. Humanized forms of the 01G06 antibody are listed below together with the amino acid sequences of their respective heavy and light chain variable regions. Exemplary humanized anti-GDF15 antibodies include: Hu01G06-1; Hu01G06-46; Hu01G06-52; Hu01G06-100; Hu01G06-101; Hu01G06-102; Hu01G06-103; Hu01G06-104; Hu01G06-105; Hu01G06-106; Hu01G06-107; Hu01G06-108; Hu01G06-109; Hu01G06-110; Hu01G06-111; Hu01G06-112; Hu01G06-113; Hu01G06-114; Hu01G06-122; Hu01G06-127; Hu01G06-135; Hu01G06-138; Hu01G06-146; Hu06C11-1; Hu06C11-27; Hu06C11-30; Hu14F11-1; Hu14F11-23; Hu14F11-24; Hu14F11-39; and Hu14F11-47. The amino acid sequences for the heavy chain and light chain for each of the aforementioned antibodies is set forth below in Table 3.

**TABLE 3**

| Antibody Name | Light Chain | Heavy Chain |
|---|---|---|
| 01G06 (murine) | SEQ ID NO:25 | SEQ ID NO:37 |
| Hu01G06-1 | SEQ ID NO:26 | SEQ ID NO:38 |
| Hu01 G06-46 | SEQ ID NO:27 | SEQ ID NO:39 |
| Hu01G06-52 | SEQ ID NO:27 | SEQ ID NO:40 |
| Hu01 G06-100 | SEQ ID NO:27 | SEQ ID NO:41 |
| Hu01G06-101 | SEQ ID NO:27 | SEQ ID NO:42 |
| Hu01 G06-102 | SEQ ID NO:27 | SEQ ID NO:43 |
| Hu01G06-103 | SEQ ID NO:27 | SEQ ID NO:44 |
| Hu01 G06-104 | SEQ ID NO:27 | SEQ ID NO:45 |
| Hu01G06-105 | SEQ ID NO:28 | SEQ ID NO:41 |
| Hu01 G06-106 | SEQ ID NO:28 | SEQ ID NO:42 |
| Hu01 G06-107 | SEQ ID NO:28 | SEQ ID NO:43 |
| Hu01 G06-108 | SEQ ID NO:28 | SEQ ID NO:44 |
| Hu01 G06-109 | SEQ ID NO:28 | SEQ ID NO:45 |
| Hu01G06-110 | SEQ ID NO:29 | SEQ ID NO:41 |
| Hu01G06-111 | SEQ ID NO:29 | SEQ ID NO:42 |
| Hu01G06-112 | SEQ ID NO:29 | SEQ ID NO:43 |
| Hu01 G06-113 | SEQ ID NO:29 | SEQ ID NO:44 |
| Hu01G06-114 | SEQ ID NO:29 | SEQ ID NO:45 |
| Hu01G06-122 | SEQ ID NO:29 | SEQ ID NO:46 |
| Hu01G06-127 | SEQ ID NO:30 | SEQ ID NO:47 |
| Hu01G06-135 | SEQ ID NO:29 | SEQ ID NO:48 |
| Hu01G06-138 | SEQ ID NO:29 | SEQ ID NO:49 |
| Hu01 G06-146 | SEQ ID NO:30 | SEQ ID NO:49 |
| 06C11 (murine) | SEQ ID NO:31 | SEQ ID NO:50 |
| Hu06C11-1 | SEQ ID NO:32 | SEQ ID NO:38 |
| Hu06C11-27 | SEQ ID NO:33 | SEQ ID NO:51 |
| Hu06C11-30 | SEQ ID NO:33 | SEQ ID NO:52 |
| 14F11 (murine) | SEQ ID NO:34 | SEQ ID NO:53 |
| Hu14F11-1 | SEQ ID NO:35 | SEQ ID NO:54 |
| Hu14F11-23 | SEQ ID NO:35 | SEQ ID NO:55 |
| Hu14F11-24 | SEQ ID NO:32 | SEQ ID NO:54 |
| Hu14F11-39 | SEQ ID NO:36 | SEQ ID NO:56 |
| Hu14F11-47 | SEQ ID NO:36 | SEQ ID NO:57 |

It is understood that the antibodies described herein can be designed, tested, and formulated using techniques known in the art.

SEQ ID NO:25

1 diqmtqspas lsasvgetvt itcrtsenlh nylawyqqkq gkspqllvyd aktladgvps
61 rfsgsgsgtq yslkinslqp edfgsyycqh fwsspytfgg gtkleikrad aaptvsifpp
121 sseqltsgga svvcflnnfy pkdinvkwki dgserqngvl nswtdqdskd stysmsstlt
181 ltkdeyerhn sytceathkt stspivksfn rnec

SEQ ID NO:26

1 diqmtqspas lsasvgetvt itcrtsenlh nylawyqqkq gkspqllvyd aktladgvps
61 rfsgsgsgtq yslkinslqp edfgsyycqh fwsspytfgg gtkleikrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:27

1 diqmtqspss lsasvgdrvt itcrtsenlh nylawyqqkp gkspkllvyd aktladgvps
61 rfsgsgsgtd ytltisslqp edfatyycqh fwsspytfgq gtkleikrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:29

1 diqmtqspss lsasvgdrvt itcrtsenlh nylawyqqkp gkapklliyd aktladgvps
61 rfsgsgsgtd ytltisslqp edfatyycqh fwsspytfgq gtkleikrtv aapsvfifpp

121 sdeqlksgta svvcllnnfy preakvqwkv dnalqsgnsq esvteqdskd styslsstlt
181 lskadyekhk vyacevthqg lsspvtksfn rgec

SEQ ID NO:28

1 diqmtqspss lsasvgdrvt itcrtsenlh nylawyqqkp gkspklliyd aktladgvps
61 rfsgsgsgtd ytltisslqp edfatyycqh fwsspytfgq gtkleikrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:32

  1 divmtqsqkf mstsvgdrvs vtckasqnvg tnvawfqqkp gqspkaliys asyrysgvpd
 61 rftgsgsgtd filtisnvqs edlaeyfcqq ynnypltfga gtklelkrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:33

1 diqmtqspss lsasvgdrvt itckasqnvg tnvawfqqkp gkapksliys asyrysgvps
61 rfsgsgsgtd ftltisslqp edfatyycqq ynnypltfgq gtkleikrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:35

  1 divmtqsqkf mstsvgdrvs vtckasqnvg tnvawyqqkp gqspkaliys psyrysgvpd
 61 rftgsgsgtd ftltisnvqs edlaeyfcqq ynsyphtfgg gtklemkrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:36

  1 diqmtqspss lsasvgdrvt itckasqnvg tnvawfqqkp gkspkaliys psyrysgvps
 61 rfsgsgsgtd ftltisslqp edfatyfcqq ynsyphtfgq gtkleikrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:37

1 evllqqsgpe lvkpgasvki pckasgytft dynmdwvkqs hgkslewigq inpnnggiff
61 nqkfkgkatl tvdkssntaf mevrsltsed tavyycarea ittvgamdyw gqgtsvtvss
121 akttppsvyp lapgsaaqtn smvtlgclvk gyfpepvtvt wnsgslssgv htfpavlqsd
181 lytlsssvtv psstwpsetv tcnvahpass tkvdkkivpr dcgckpcict vpevssvfif
241 ppkpkdvlti tltpkvtcvv vdiskddpev qfswfvddve vhtaqtqpre eqfnstfrsv
301 selpimhqdw lngkefkcrv nsaafpapie ktisktkgrp kapqvytipp pkeqmakdkv
361 sltcmitdff peditvewqw ngqpaenykn tqpimdtdgs yfvysklnvq ksnweagntf
421 tcsvlheglh nhhtekslsh spgk

SEQ ID NO:30

1 diqmtqspss lsasvgdrvt itcrtsenlh nylawyqqkp gkspklliyd aktladgvps

61 rfsgsgsgtd ytltisslqp edfatyycqh fwsdpytfgq gtkleikrtv aapsvfifpp
121 sdeqlksgta svvcllnnfy preakvqwkv dnalqsgnsq esvteqdskd styslsstlt
181 lskadyekhk vyacevthqg lsspvtksfn rgec

SEQ ID NO:38

1 evllqqsgpe lvkpgasvki pckasgytft dynmdwvkqs hgkslewigq inpnnggiff
61 nqkfkgkatl tvdkssntaf mevrsltsed tavyycarea ittvgamdyw gqgtsvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:39

1 qvqlvqsgae vkkpgasvkv sckasgytft dynmdwvrqa pgkslewigq inpnnggiff
61 nqkfkgratl tvdtstntay melrslrsdd tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:40

1 qvqlvqsgae vkkpgssvkv sckasgytft dynmdwvrqa pgkslewigq inpnnggiff
61 nqkfkgratl tvdkstntay melsslrsed tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:41

  1 qvqlvqsgae vkkpgasvkv sckasgytft dynmdwvrqa pgqglewmgq inpnnggiff
 61 nqkfkgrvtl ttdtststay melrslrsdd tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:43

1 qvqlvqsgae vkkpgasvkv sckasgytft dynmdwvrqa pgqslewmgq inpnnggiff
61 nqkfqgrvtl ttdtststay melrslrsdd tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg

241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:42

  1 qvqlvqsgae vkkpgasvkv sckasgytft dynmdwvrqa pgqglewmgq inpnnggiff
 61 nqkfqgrvtl ttdtststay melrslrsdd tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:44

  1 qvqlvqsgae vkkpgssvkv sckasgytfs dynmdwvrqa pgqglewmgq inpnnggiff
 61 nqkfkgrvtl tadkststay melsslrsed tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:45

1 qvqlvqsgae vkkpgssvkv sckasgytfs dynmdwvrqa pgqglewmgq inpnnggiff
61 nqkfqgrvtl tadkststay melsslrsed tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:46

1 qvqlvqsgae vkkpgasvkv sckasgytft dynmdwvrqa pgqslewmgq inpynhliff
61 nqkfqgrvtl ttdtststay melrslrsdd tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:47

1 qvqlvqsgae vkkpgasvkv sckasgytft dynmdwvrqa pgqslewmgq inpnngliff
61 nqkfqgrvtl ttdtststay melrslrsdd tavyycarea ittvgamdyw gqgtlvtvss
121 astkgpsvfp lapsskstsg gtaalgclvk dyfpepvtvs wnsgaltsgv htfpavlqss

181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:48

1 qvqlvqsgae vkkpgssvkv sckasgytfs dynmdwvrqa pgqglewmgq inpnngliff
61 nqkfkgrvtl tadkststay melsslrsed tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:49

1 qvqlvqsgae vkkpgssvkv sckasgytfs dynmdwvrqa pgqglewmgq inpynhliff
61 nqkfkgrvtl tadkststay melsslrsed tavyycarea ittvgamdyw gqgtlvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:38

1 evllqqsgpe lvkpgasvki pckasgytft dynmdwvkqs hgkslewigq inpnnggiff
61 nqkfkgkatl tvdkssntaf mevrsltsed tavyycarea ittvgamdyw gqgtsvtvss
121 astkgpsvfp lapsskstsg gtaalgclvk dyfpepvtvs wnsgaltsgv htfpavlqss
181 glyslssvvt vpssslgtqt yicnvnhkps ntkvdkrvep kscdkthtcp pcpapellgg
241 psvflfppkp kdtlmisrtp evtcvvvdvs hedpevkfnw yvdgvevhna ktkpreeqyn
301 styrvvsvlt vlhqdwlngk eykckvsnka lpapiektis kakgqprepq vytlppsree
361 mtknqvsltc lvkgfypsdi avewesngqp ennykttppv ldsdgsffly skltvdksrw
421 qqgnvfscsv mhealhnhyt qkslslspgk

SEQ ID NO:51

1 qvtlkesgpa lvkptqtltl tctfsgfsln tygmgvswir qppgkalewl ahiywdddkr
61 ynpslktrlt iskdtsknqv vltitnvdpv dtavyycaqr gyddywgywg qgtlvtissa
121 stkgpsvfpl apsskstsgg taalgclvkd yfpepvtvsw nsgaltsgvh tfpavlqssg
181 lyslssvvtv pssslgtqty icnvnhkpsn tkvdkrvepk scdkthtcpp cpapellggp
241 svflfppkpk dtlmisrtpe vtcvvvdvsh edpevkfnwy vdgvevhnak tkpreeqyns
301 tyrvvsvltv lhqdwlngke ykckvsnkal papiektisk akgqprepqv ytlppsreem
361 tknqvsltcl vkgfypsdia vewesngqpe nnykttppvl dsdgsfflys kltvdksrwq
421 qgnvfscsvm healhnhytq kslslspgk

SEQ ID NO:52

1 qvtlkesgpt lvkptqtltl tctfsgfsln tygmgvswir qppgkglewl ahiywdddkr
61 ynpslksrlt itkdtsknqv vltitnmdpv dtatyycaqr gyddywgywg qgtlvtvssa

121 stkgpsvfpl apsskstsgg taalgclvkd yfpepvtvsw nsgaltsgvh tfpavlqssg
181 lyslssvvtv pssslgtqty icnvnhkpsn tkvdkrvepk scdkthtcpp cpapellggp
241 svflfppkpk dtlmisrtpe vtcvvvdvsh edpevkfnwy vdgvevhnak tkpreeqyns
301 tyrvvsvltv lhqdwlngke ykckvsnkal papiektisk akgqprepqv ytlppsreem
361 tknqvsltcl vkgfypsdia vewesngqpe nnykttppvl dsdgsfflys kltvdksrwq
421 qgnvfscsvm healhnhytq kslslspgk

SEQ ID NO:54

1 qvtlkesgpg ilqpsqtlsl tcsfsgfsls tygmgvgwir qpsgkglewl adiwwdddky
61 ynpslksrlt iskdtssnev flkiaivdta dtatyycarr ghysamdywg qgtsvtvssa
121 stkgpsvfpl apsskstsgg taalgclvkd yfpepvtvsw nsgaltsgvh tfpavlqssg
181 lyslssvvtv pssslgtqty icnvnhkpsn tkvdkrvepk scdkthtcpp cpapellggp
241 svflfppkpk dtlmisrtpe vtcvvvdvsh edpevkfnwy vdgvevhnak tkpreeqyns
301 tyrvvsvltv lhqdwlngke ykckvsnkal papiektisk akgqprepqv ytlppsreem
361 tknqvsltcl vkgfypsdia vewesngqpe nnykttppvl dsdgsfflys kltvdksrwq
421 qgnvfscsvm healhnhytq kslslspgk

SEQ ID NO:55

1 qvtlkesgpg ilqpsqtlsl tcsfsgfsln tygmgvswir qpsgkglewl ahiywdddkr
61 ynpslksrlt iskdasnnrv flkitsvdta dtatyycaqr gyddywgywg qgtlvtisaa
121 stkgpsvfpl apsskstsgg taalgclvkd yfpepvtvsw nsgaltsgvh tfpavlqssg
181 lyslssvvtv pssslgtqty icnvnhkpsn tkvdkrvepk scdkthtcpp cpapellggp
241 svflfppkpk dtlmisrtpe vtcvvvdvsh edpevkfnwy vdgvevhnak tkpreeqyns
301 tyrvvsvltv lhqdwlngke ykckvsnkal papiektisk akgqprepqv ytlppsreem
361 tknqvsltcl vkgfypsdia vewesngqpe nnykttppvl dsdgsfflys kltvdksrwq
421 qgnvfscsvm healhnhytq kslslspgk

SEQ ID NO:56

  1 qitlkesgpt lvkptqtltl tctfsgfsls tygmgvgwir qppgkalewl adiwwdddky
 61 ynpslksrlt itkdtsknqv vltmtnmdpv dtatyycarr ghysamdywg qgtlvtvssa
121 stkgpsvfpl apsskstsgg taalgclvkd yfpepvtvsw nsgaltsgvh tfpavlqssg
181 lyslssvvtv pssslgtqty icnvnhkpsn tkvdkrvepk scdkthtcpp cpapellggp
241 svflfppkpk dtlmisrtpe vtcvvvdvsh edpevkfnwy vdgvevhnak tkpreeqyns
301 tyrvvsvltv lhqdwlngke ykckvsnkal papiektisk akgqprepqv ytlppsreem
361 tknqvsltcl vkgfypsdia vewesngqpe nnykttppvl dsdgsfflys kltvdksrwq
421   nvfscsvm healhnhytq kslslspgk

SEQ ID NO:57

1 qvtlkesgpa lvkptqtltl tctfsgfsls tygmgvgwir qppgkalewl adiwwdddky
61 ynpslksrlt iskdtsknqv vltmtnmdpv dtavyycarr ghysamdywg qgtlvtvssa
121 stkgpsvfpl apsskstsgg taalgclvkd yfpepvtvsw nsgaltsgvh tfpavlqssg
181 lyslssvvtv pssslgtqty icnvnhkpsn tkvdkrvepk scdkthtcpp cpapellggp
241 svflfppkpk dtlmisrtpe vtcvvvdvsh edpevkfnwy vdgvevhnak tkpreeqyns
301 tyrvvsvltv lhqdwlngke ykckvsnkal papiektisk akgqprepqv ytlppsreem
361 tknqvsltcl vkgfypsdia vewesngqpe nnykttppvl dsdgsfflys kltvdksrwq
421 qgnvfscsvm healhnhytq kslslspgk

SEQ ID NO:50

1 qvtlkesgpg ilqpsqtlsl tcsfsgfsln tygmgvswir qpsgkglewl ahiywdddkr

61 ynpslksrlt iskdasnnrv flkitsvdta dtatyycaqr gyddywgywg qgtlvtisaa
121 kttppsvypl apgsaaqtns mvtlgclvkg yfpepvtvtw nsgslssgvh tfpavlqsdl
181 ytlsssvtvp sstwpsetvt cnvahpasst kvdkkivprd cgckpcictv pevssvfifp
241 pkpkdvltit ltpkvtcvvv diskddpevq fswfvddvev htaqtqpree qfnstfrsvs
301 elpimhqdwl ngkefkcrvn saafpapiek tisktkgrpk apqvytippp keqmakdkvs
361 ltcmitdffp editvewqwn gqpaenyknt qpimdtdgsy fvysklnvqk snweagntft
421 csvlheglhn hhtekslshs pgk

SEQ ID NO:31

1 divmtqsqkf mstsvgdrvs vtckasqnvg tnvawfqqkp gqspkaliys asyrysgvpd
61 rftgsgsgtd filtisnvqs edlaeyfcqq ynnypltfga gtklelkrad aaptvsifpp
121 sseqltsgga svvcflnnfy pkdinvkwki dgserqngvl nswtdqdskd stysmsstlt
181 ltkdeyerhn sytceathkt stspivksfn rnec

SEQ ID NO:53

1 qvtlkesgpg ilqpsqtlsl tcsfsgfsls tygmgvgwir qpsgkglewl adiwwdddky
61 ynpslksrlt iskdtssnev flkiaivdta dtatyycarr ghysamdywg qgtsvtvssa
121 kttppsvypl apgsaaqtns mvtlgclvkg yfpepvtvtw nsgslssgvh tfpavlqsdl
181 ytlsssvtvp sstwpsetvt cnvahpasst kvdkkivprd cgckpcictv pevssvfifp
241 pkpkdvltit ltpkvtcvvv diskddpevq fswfvddvev htaqtqpree qfnstfrsvs
301 elpimhqdwl ngkefkcrvn saafpapiek tisktkgrpk apqvytippp keqmakdkvs
361 ltcmitdffp editvewqwn gqpaenyknt qpimdtdgsy fvysklnvqk snweagntft
421 csvlheglhn hhtekslshs pgk

SEQ ID NO:34

1 divmtqsqkf mstsvgdrvs vtckasqnvg tnvawyqqkp gqspkaliys psyrysgvpd
61 rftgsgsgtd ftltisnvqs edlaeyfcqq ynsyphtfgg gtklemkrad aaptvsifpp
121 sseqltsgga svvcflnnfy pkdinvkwki dgserqngvl nswtdqdskd stysmsstlt
181 ltkdeyerhn sytceathkt stspivksfn rnec

The antibody may be a neutralizing antibody, which reduces GDF15 activity. For example, the antibody may reduce GDF15 activity in an *in vivo* assay (see, *e.g.,* Johnen et al., 2007, NATURE MEDICINE 13:1333-1340) by at least 10%, preferably 20%, 30% or 40%, and more preferably at least about 50%, 60%, 80% or 90% of GDF15 compared to GDF15 activity measured in the same assay under the same conditions in the absence of the antibody. The antibody may selectively and/or significantly reduce or inhibit the binding of GDF15 to its endogenous receptor. As used herein, the term "significantly reduces or inhibits binding" of GDF15 to its receptor is understood to mean that the antibody inhibits GDF15 binding with a potency or percent inhibition that measures at least 10%, preferably 20%, 30% or 40%, and more preferably at least about 50%, 60%, 80% or 90% of GDF15 [serum level/activity] in the absence of said antibody. Binding can be measured using a direct or sandwich enzyme-linked immunosorbent assay (ELISA), as described, *e.g.,* in Tsai et al., 2013, PLOS ONE, 8:e55174. As used herein, the term "selectively" in the context of an antibody that binds to GDF15 or GDF15 receptor is understood to mean that the antibody binds GDF15 or a GDF15 receptor with a binding affinity that is at least two, three, four, five or ten times greater than that of a functionally unrelated protein or another member of the TGF-β superfamily or a receptor of a member of the TGF-β superfamily.

Methods for reducing or eliminating the antigenicity of antibodies and antibody fragments are known in the art. When the antibodies are to be administered to a human, the antibodies preferably are "humanized" to reduce or eliminate antigenicity in humans. Preferably, each humanized antibody has the same or substantially the same affinity for the antigen as the non-humanized mouse antibody from which it was derived.

In one humanization approach, chimeric proteins are created in which mouse immunoglobulin constant regions are replaced with human immunoglobulin constant regions. See, *e.g.,* Morrison et al., 1984, PROC. NAT. ACAD. SCI. 81:6851-6855, Neuberger et al., 1984, NATURE 312:604-608; U.S. Patent Nos. 6,893,625 (Robinson); 5,500,362 (Robinson); and 4,816,567 (Cabilly).

In an approach known as CDR grafting, the CDRs of the light and heavy chain variable regions are grafted into frameworks from another species. For example, murine CDRs can be grafted into human FRs. In some embodiments, the CDRs of the light and heavy chain variable regions of an anti-GDF 15 antibody are grafted into human FRs or consensus human FRs. To create consensus human FRs, FRs from several human heavy chain or light chain amino acid sequences are aligned to identify a consensus amino acid sequence. CDR grafting is described in U.S. Patent Nos. 7,022,500 (Queen); 6,982,321 (Winter); 6,180,370 (Queen); 6,054,297 (Carter); 5,693,762 (Queen); 5,859,205 (Adair); 5,693,761 (Queen); 5,565,332 (Hoogenboom); 5,585,089 (Queen); 5,530,101 (Queen); Jones etal., 1986, NATURE 321: 522-525; Riechmann et al., 1988, NATURE 332: 323-327; Verhoeyen et al., 1988, SCIENCE 239: 1534-1536; and Winter, 1998, FEBS LETT 430: 92-94.

In an approach called "SUPERHUMANIZATION™," human CDR sequences are chosen from human germline genes, based on the structural similarity of the human CDRs to those of the mouse antibody to be humanized. See, *e.g.,* U.S. Patent No. 6,881,557 (Foote); and Tan et al., 2002, J. IMMUNOL. 169:1119-1125.

Other methods to reduce immunogenicity include "reshaping," "hyperchimerization," and "veneering/resurfacing." See, *e.g.,* Vaswami et al., 1998, ANNALS OF ALLERGY, ASTHMA, & IMMUNOL. 81:105; Roguska et al., 1996, PROT. ENGINEER 9:895-904; and U.S. Patent No. 6,072,035 (Hardman). In the veneering/resurfacing approach, the surface accessible amino acid residues in the murine antibody are replaced by amino acid residues more frequently found at the same positions in a human antibody. This type of antibody resurfacing is described, *e.g*., in U.S. Patent No. 5,639,641 (Pedersen).

Another approach for converting a mouse antibody into a form suitable for medical use in humans is known as ACTIVMAB™ technology (Vaccinex, Inc., Rochester, NY), which involves a vaccinia virus-based vector to express antibodies in mammalian cells. High levels of combinatorial diversity of IgG heavy and light chains are said to be produced. See, *e.g.,* U.S. Patent Nos. 6,706,477 (Zauderer); 6,800,442 (Zauderer); and 6,872,518 (Zauderer).

Another approach for converting a mouse antibody into a form suitable for use in humans is technology practiced commercially by KaloBios Pharmaceuticals, Inc. (Palo Alto, CA). This technology involves the use of a proprietary human "acceptor" library to produce an "epitope focused" library for antibody selection.

Another approach for modifying a mouse antibody into a form suitable for medical use in humans is HUMAN ENGINEERING™ technology, which is practiced commercially by XOMA (US) LLC. See, *e.g.,* PCT Publication No. WO 93/11794 and U.S. Patent Nos. 5,766,886 (Studnicka); 5,770,196 (Studnicka); 5,821,123 (Studnicka); and 5,869,619 (Studnicka).

Any suitable approach, including any of the above approaches, can be used to reduce or eliminate human immunogenicity of an antibody.

In addition, it is possible to create fully human antibodies in mice. Fully human mAbs lacking any non-human sequences can be prepared from human immunoglobulin transgenic mice by techniques referenced in, *e.g.,* Lonberg et al., NATURE 368:856-859, 1994; Fishwild et al., NATURE BIOTECHNOLOGY 14:845-851, 1996; and Mendez et al., NATURE GENETICS 15:146-156, 1997. Fully human mAbs can also be prepared and optimized from phage display libraries by techniques referenced in, *e.g.,* Knappik et al., J. MOL. BIOL. 296:57-86, 2000; and Krebs et al., J. Immunol. Meth. 254:67-84 2001).

It is contemplated that variants and derivatives of GDF15 that act as decoys can be useful in the practice of the invention. For example, through deletion analysis, it may be possible to identify smaller biologically active fragments of GDF15 that compete with endogenous GDF15 for its cognate receptor. Similarly, it is possible to create soluble biologically active fragments of the GDF15 receptor that compete with endogenous GDF15 receptor for available GDF. For example, "biologically active fragments" include, but are not limited to, fragments of a naturally-occurring GDF15 (or homolog) or a GDF15 receptor (or homolog) that compete with endogenous GDF15 or an endogenous GDF15 receptor, respectively, for binding to a cognate binding partner (*e.g*., GDF15 receptor or GDF15, respectively).

It is contemplated that antisense nucleic acids (DNA and RNA) and small interfering nucleic acids (*e.g*., siRNAs) can be designed and used using techniques known in the art. Exemplary siRNA inhibitors of GDF15 include siRNAs from Santa Cruz Biotech (Catalog No. sc-39799, targeting mouse GDF15; and Catalog No. sc-39798, targeting human GDF15), siRNAs from Life Technologies (Cat. Nos. AM16708, 4392420, and 1299001, targeting human GDF15; and Cat. Nos. 1320001 and 4390771, targeting mouse GDF15; and Cat. Nos. 1330001 and 4390771, targeting rat GDF15), siRNAs from Fisher Scientific (Catalog No. NC0683807, targeting human GDF15), siRNAs from Origene (Catalog No. SR306321, targeting human GDF15), siRNAs from amsbio (Catalog No. SR509800, targeting rate GDF15), siRNAs from Dharmacon (including Catalog No. D-019875-02, targeting human GDF15), siRNAs from Sigma-Aldrich (Catalog No. EHU052901, targeting human GDF15), and siRNAs described in Kim et a/., 2005, MOLECULAR CANCER THERAPEUTICS, 4:487-493, Chang et al., 2007, MOL. CANCER THERAPEUTICS, 6:2271-2279, and Boyle et al., 2009, J. INVEST. DERMATOL., 129:383-391.

### II. Formulation and Delivery of GDF15 Modulators

Pharmaceutical compositions containing GDF15 modulators, such as those disclosed herein, can be formulated into dosage forms or dosage units using standard formulation techniques. However, the pharmaceutical composition should be formulated to be compatible with its intended route of administration.

The compositions described herein can be administered to a subject via any route, including, but not limited to, intravenous (*e.g*., by infusion pumps), intraperitoneal, intraocular, intra-arterial, intrapulmonary, oral, inhalation, intravesicular, intramuscular, intra-tracheal, subcutaneous, intraocular, intrathecal, transdermal, transpleural, intraarterial, topical, inhalational (*e.g*., as mists of sprays), mucosal (such as via nasal mucosa), subcutaneous, transdermal, gastrointestinal, intraarticular, intracistemal, intraventricular, rectal (*i.e.,* via suppository), vaginal (*i.e.,* via pessary), intracranial, intraurethral, intrahepatic, and intratumoral. The compositions may be administered systemically (for example by intravenous injection). The compositions may be administered locally (for example by intraarterial or intraocular injection). A preferred route of administration for GDF15 modulators, such as an antibody, is via intravenous infusion.

Useful formulations can be prepared by methods well known in the pharmaceutical art. For example, see REMINGTON'S PHARMACEUTICAL SCIENCES, 18th ed. (Mack Publishing Company, 1990). Formulation components suitable for parenteral administration include a sterile diluent such as bacteriostatic water for injection, physiological saline, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as EDTA; buffers such as acetates, citrates or phosphates; and agents for the adjustment of tonicity such as sodium chloride or dextrose. The carrier should be stable under the conditions of manufacture and storage, and should be preserved against microorganisms. The antibody may be lyophilized, and then reconstituted in buffered saline, at the time of administration.

For therapeutic use, an antibody preferably is combined with a pharmaceutically acceptable carrier. As used herein, "pharmaceutically acceptable carrier" means buffers, carriers, and excipients suitable for use in contact with the tissues of human beings and animals without excessive toxicity, irritation, allergic response, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration. The use of such media and agents for pharmaceutically active substances is known in the art.

The pharmaceutical compositions preferably are sterile. Sterilization can be accomplished, for example, by filtration through sterile filtration membranes. Where the composition is lyophilized, filter sterilization can be conducted prior to or following lyophilization and reconstitution.

Generally, a therapeutically effective amount of active component is in the range of 0.1 mg/kg to 100 mg/kg, *e.g.,* 1 mg/kg to 100 mg/kg, 1 mg/kg to 10 mg/kg. The amount administered will depend on variables such as the type and extent of disease or indication to be treated, the overall health of the patient, the *in vivo* potency of the antibody, the pharmaceutical formulation, and the route of administration. The initial dosage can be increased beyond the upper level in order to rapidly achieve the desired blood-level or tissue-level. Alternatively, the initial dosage can be smaller than the optimum, and the daily dosage may be progressively increased during the course of treatment. Human dosage can be optimized, *e.g*., in a conventional Phase I dose escalation study designed to run from 0.5 mg/kg to 20 mg/kg. Dosing frequency can vary, depending on factors such as route of administration, dosage amount, serum half-life of the antibody, and the disease being treated. Exemplary dosing frequencies are once per day, once per week and once every two weeks.

The optimal effective amount of the compositions can be determined empirically and will depend on the type and severity of the disease, route of administration, disease progression and health, mass and body area of the subject. Such determinations are within the skill of one in the art. Examples of dosages of GDF15 modulator molecules which can be used for methods described herein include, but are not limited to, an effective amount within the dosage range of any of about 0.01 µg/kg to about 300 mg/kg, or within about 0.1 µg/kg to about 40 mg/kg, or with about 1 µg/kg to about 20 mg/kg, or within about 1 µg/kg to about 10 mg/kg. For example, when administered subcutaneously, the composition may be administered at low microgram ranges, including for example about 0.1 µg/kg or less, about 0.05 µg/kg or less, or 0.01 µg/kg or less.

The amount of GDF15 modulators administered to a subject may be about 10 µg to about 500 mg per dose, including for example any of about 10 µg to about 50 µg, about 50 µg to about 100 µg, about 100 µg to about 200 µg, about 200 µg to about 300 µg, about 300 µg to about 500 µg, about 500 µg to about 1 mg, about 1 mg to about 10 mg, about 10 mg to about 50 mg, about 50 mg to about 100 mg, about 100 mg to about 200 mg, about 200 mg to about 300 mg, about 300 mg to about 400 mg, or about 400 mg to about 500 mg per dose. The GDF15 modulator may be administered at a dose from about 0.025 mg to about 4 mg, from about 0.035 mg to about 2 mg, from about 0.05 mg to about 2 mg, from about 0.1 mg to about 2 mg, from about 0.2 mg to about 1 mg, or from about 0.2 mg to about 0.8 mg of the GDF15 modulator can be administered. A dose of 0.5 mg of GDF15 modulator may be administered locally. A dose of from about 0.05 mg to about 2 mg, from about 0.2 mg to about 2 mg, from about 0.05 mg to about 1.5 mg, from about 0.15 mg to about 1.5 mg, from about 0.4 mg to about 1 mg, or from about 0.5 mg to about 0.8 mg of GDF15 modulator may be administered locally.

The GDF15 modulator compositions may be administered in a single daily dose, or the total daily dose may be administered in divided dosages of two, three, or four times daily. The compositions can also be administered less frequently than daily, for example, six times a week, five times a week, four times a week, three times a week, twice a week, once a week, once every two weeks, once every three weeks, once a month, once every two months, once every three months, or once every six months. The compositions may also be administered in a sustained release formulation, such as in an implant which gradually releases the composition for use over a period of time, and which allows for the composition to be administered less frequently, such as once a month, once every 2-6 months, once every year, or even a single administration. The sustained release devices (such as pellets, nanoparticles, microparticles, nanospheres, microspheres, and the like) may be administered by injection or surgical implanted in various locations in the body.

The dosing of the GDF15 modulator may be titrated such that the dose is sufficient to reduce or prevent adverse effects, but yet fully or partially inhibit the activity of the GDF15.

In some aspects, the activity of GDF15 can be modulated in a target cell using antisense nucleic acids or small interfering nucleic acids. Modulation can be achieved using expression constructs known in the art, *e.g*., naked DNA constructs, DNA vector based constructs, and/or viral vector and/or viral based constructs to express nucleic acids encoding an anti-GDF15 siRNA or antisense molecule.

Exemplary DNA constructs and the therapeutic use of such constructs are well known to those of skill in the art (see, *e.g.,* Chiarella et al., 2008, RECENT PATENTS ANTI-INFECT. DRUG DISC., 3:93-101; Gray et al., 2008, EXPERT OPIN. BIOL. THER., 8:911-922; Melman et al., 2008, HUM. GENE THER., 17:1165-1176). Naked DNA constructs typically include one or more therapeutic nucleic acids (*e.g*., GDF15 modulators) and a promoter sequence. A naked DNA construct can be a DNA vector, commonly referred to as pDNA. Naked DNA typically do not integrate into chromosomal DNA. Generally, naked DNA constructs do not require, or are not used in conjunction with, the presence of lipids, polymers, or viral proteins. Such constructs may also include one or more of the non-therapeutic components described herein.

DNA vectors are known in the art and typically are circular double stranded DNA molecules. DNA vectors usually range in size from three to five kilo-base pairs (*e.g*., including inserted therapeutic nucleic acids). Like naked DNA, DNA vectors can be used to deliver and express one or more therapeutic proteins in target cells. DNA vectors do not integrate into chromosomal DNA.

Generally, DNA vectors include at least one promoter sequence that allows for replication in a target cell. Uptake of a DNA vector may be facilitated by combining the DNA vector with, for example, a cationic lipid, and forming a DNA complex. Typically, viral vectors are double stranded circular DNA molecules that are derived from a virus. Viral vectors typically are larger in size than naked DNA and DNA vector constructs and have a greater capacity for the introduction of foreign (*i.e.,* not virally encoded) genes. Like naked DNA and DNA vectors, viral vectors can be used to deliver and express one or more therapeutic nucleic acids in target cells. Unlike naked DNA and DNA vectors, certain viral vectors stably incorporate themselves into chromosomal DNA. Typically, viral vectors include at least one promoter sequence that allows for replication of one or more vector encoded nucleic acids, *e.g.*, a therapeutic nucleic acid, in a host cell. Viral vectors may optionally include one or more non-therapeutic components described herein. Advantageously, uptake of a viral vector into a target cell does not require additional components, *e.g*., cationic lipids. Rather, viral vectors transfect or infect cells directly upon contact with a target cell.

The approaches described herein include the use of retroviral vectors, adenovirus-derived vectors, and/or adeno-associated viral vectors as recombinant gene delivery systems for the transfer of exogenous genes *in vivo,* particularly into humans. Protocols for producing recombinant retroviruses and for infecting cells *in vitro* or *in vivo* with such viruses can be found in CURRENT PROTOCOLS IN MOLECULAR BIOLOGY, Ausubel, F. M. et al. (eds.) Greene Publishing Associates, 1989, Sections 9.10-9.14, and other standard laboratory manuals.

Viruses that are used as transduction agents of DNA vectors and viral vectors such as adenoviruses, retroviruses, and lentiviruses may be used in practicing the present invention. Illustrative retroviruses include, but are not limited to: Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus. As used herein, the term "lentivirus" refers to a group (or genus) of complex retroviruses. Illustrative lentiviruses include, but are not limited to: HIV (human immunodeficiency virus; including HIV type 1, and HIV type 2); visna-maedi virus (VMV) virus; the caprine arthritis-encephalitis virus (CAEV); equine infectious anemia virus (EIAV); feline immunodeficiency virus (FIV); bovine immune deficiency virus (BIV); and simian immunodeficiency virus (SIV).

An adenovirus can be used in accordance with the methods described herein. The genome of an adenovirus can be manipulated such that it encodes and expresses a gene product of interest but is inactivated in terms of its ability to replicate in a normal lytic viral life cycle. Suitable adenoviral vectors derived from the adenovirus strain Ad type 5 dl324 or other strains of adenovirus (*e.g*., Ad2, Ad3, Ad7 etc.) are known to those skilled in the art. Recombinant adenoviruses can be advantageous in certain circumstances in that they are not capable of infecting nondividing cells and can be used to infect a wide variety of cell types, including epithelial cells Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis *in situ* where introduced DNA becomes integrated into the host genome (*e.g*., retroviral DNA). Moreover, the carrying capacity of the adenoviral genome for foreign DNA is large (up to 8 kilobases) relative to other gene delivery vectors.

Adeno-associated virus is a naturally occurring defective virus that requires another virus, such as an adenovirus or a herpes virus, as a helper virus for efficient replication and a productive life cycle. It is also one of the few viruses that may integrate its DNA into nondividing cells, and exhibits a high frequency of stable integration.

One or more viral vectors that expresses a therapeutic transgene or transgenes encoding a GDF15 modulator may be administered by direct injection to a cell, tissue, or organ of a subject, *in vivo.* Cells may be transduced *in vitro* or *ex vivo* with such a vector encapsulated in a virus, and optionally expanded *ex vivo.* The transduced cells may then be administered to the subject. Cells suitable for transduction include, but are not limited to stem cells, progenitor cells, and differentiated cells. The transduced cells may be embryonic stem cells, bone marrow stem cells, umbilical cord stem cells, placental stem cells, mesenchymal stem cells, neural stem cells, liver stem cells, pancreatic stem cells, cardiac stem cells, kidney stem cells, or hematopoietic stem cells.

Host cells transduced with viral vector of the invention that expresses one or more polypeptides, may be administered to a subject to treat chemotherapy-induced cachexia. Other methods relating to the use of viral vectors which may be utilized can be found in, *e.g*., Kay, 1997, CHEST, 111(6 Supp.):138S-142S; Ferry et al., 1998, HUM. GENE THER., 9:1975-81; Shiratory et al., 1999, LIVER, 19:265-74; Oka et al., 2000, CURR. OPIN. LIPIDOL., 11:179-86; Thule et al., 2000, GENE THER., 7: 1744-52; Yang, 1992, CRIT. REV. BIOTECHNOL., 12:335-56; Alt, 1995, J. HEPATOL., 23:746-58; Brody et al., 1994, ANN. N. Y. ACAD. SCI., 716:90-101; Strayer, 1999, EXPERT OPIN. INVESTIG. DRUGS, 8:2159-2172; Smith-Arica et a/., 2001, CURR. CARDIOL. REP., 3:43-49; and Lee et al., 2000, NATURE, 408:483-8.

Certain aspects of the invention provide conditional expression of a polynucleotide of interest. For example, expression is controlled by subjecting a cell, tissue, organism, *etc.,* to a treatment or condition that causes the polynucleotide to be expressed or that causes an increase or decrease in expression of the polynucleotide encoded by the polynucleotide of interest. Illustrative examples of inducible promoters/systems include, but are not limited to, steroid-inducible promoters such as promoters for genes encoding glucocorticoid or estrogen receptors (inducible by treatment with the corresponding hormone), metallothionine promoter (inducible by treatment with various heavy metals), MX-1 promoter (inducible by interferon), the "GeneSwitch" mifepristone-regulatable system (Sirin et al., 2003, GENE, 323:67), the cumate inducible gene switch (WO 2002/088346), tetracycline-dependent regulatory systems, etc.

Conditional expression can also be achieved by using a site specific DNA recombinase. The vector may comprises at least one (typically two) site(s) for recombination mediated by a site specific recombinase. As used herein, the terms "recombinase" or "site specific recombinase" include excisive or integrative proteins, enzymes, co-factors or associated proteins that are involved in recombination reactions involving one or more recombination sites (*e.g*., two, three, four, five, seven, ten, twelve, fifteen, twenty, thirty, fifty, etc.), which may be wild-type proteins (see Landy, 1993, CURRENT OPINION IN BIOTECHNOLOGY, 3:699-707), or mutants, derivatives (*e.g*., fusion proteins containing the recombination protein sequences or fragments thereof), fragments, and variants thereof. Illustrative examples of recombinases suitable for use include, but are not limited to: Cre, Int, IHF, Xis, Flp, Fis, Hin, Gin, OC31 , Cin, Tn3 resolvase, TndX, XerC, XerD, TnpX, Hjc, Gin, SpCCE1. and ParA.

The vectors may comprise one or more recombination sites for any of a wide variety of site specific recombinases. It is to be understood that the target site for a site specific recombinase is in addition to any site(s) required for integration of a vector (*e.g*., a retroviral vector or lentiviral vector).

The vectors may comprise a selection gene, also termed a selectable marker. Typical selection genes encode proteins that (a) confer resistance to antibiotics or other toxins, *e.g*., ampicillin, neomycin, hygromycin, methotrexate, Zeocin, Blastocidin, or tetracycline, (b) complement auxotrophic deficiencies, or (c) supply critical nutrients not available from complex media, *e.g*., the gene encoding D-alanine racemase for Bacilli. Any number of selection systems may be used to recover transformed cell lines. These include, but are not limited to, the herpes simplex virus thymidine kinase (Wigler et al., 1977, CELL, 11:223-232) and adenine phosphoribosyltransferase (Lowy et al., 1990, CELL, 22:817-823) genes which can be employed in tk- or aprt- cells, respectively.

All the molecular biological techniques required to generate an expression construct described herein are standard techniques that will be appreciated by one of skill in the art.

DNA delivery may occur parenterally, intravenously, intramuscularly, or even intraperitoneally as described, for example, in U.S. Patent Nos. 5,543,158; 5,641,515; and 5,399,363 . Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

DNA delivery may occur by use of liposomes, nanocapsules, microparticles, microspheres, lipid particles, vesicles, optionally mixing with cell penetrating polypeptides, and the like, for the introduction of the compositions of the present invention into suitable host cells. In particular, the compositions of the present invention may be formulated for delivery either encapsulated in a lipid particle, a liposome, a vesicle, a nanosphere, a nanoparticle or the like. The formulation and use of such delivery vehicles can be carried out using known and conventional techniques.

Exemplary formulations for *ex vivo* DNA delivery may also include the use of various transfection agents known in the art, such as calcium phosphate, electroporation, heat shock and various liposome formulations (*i.e.,* lipid-mediated transfection). Other suitable formulations, are well known in the pharmaceutical art, and are described, for example, in REMINGTON: THE SCIENCE AND PRACTICE OF PHARMACY, 20th Edition. Baltimore, MD: Lippincott Williams & Wilkins, 2000.

GDF15 activity may be inhibited by contacting a body fluid with a composition comprising a GDF15 modulator *ex vivo* under conditions that permit the GDF15 modulators to reduce or inhibit GDF15 activity. Suitable body fluids include those that can be returned to the individual, such as blood, plasma, or lymph. Affinity adsorption apheresis is described generally in Nilsson et al., 1988, BLOOD, 58(1):38-44; Christie et al., 1993, TRANSFUSION, 33:234-242; Richter et al., 1997, ASAIO J., 43(1):53-59; Suzuki et al., 1994, AUTOIMMUNITY, 19: 105-112; U.S. Pat. No. 5,733,254; Richter et al., 1993, METABOL. CLIN. EXP., 42:888-894; and Wallukat et al., 1996, INT'L J. CARD., 54:1910195.

Accordingly, the disclosure includes compositions comprising a GDF15 modulator for the use in method of treating one or more diseases described herein in a subject the methods comprising treating the subject's blood extracoporeally *(i.e.,* outside the body or *ex vivo*) under conditions that permit the modulator to reduce or inhibit GDF15 activity in the blood of the subject.

### III. Medical Uses

The prevention and/or reversal of cachexia, such as cancer anorexia-cachexia syndrome, by use of a GDF15 modulator in combination with anti-cancer treatment is particularly useful in cases where the anti-cancer agent itself may induce or contribute to wasting conditions in the subject being treated. Examples of anti-cancer agents whose treatment and effects can benefit from combination with one or more GDF15 modulators are platinum-based therapeutics such as cisplatin, carboplatin and oxaliplatin. Other anti-cancer agents whose treatment and effects can benefit from combination with one or more GDF15 modulators include: capecitabine, doxorubicin, and gemcitabine.

The uses of the present invention may also be for enhanced therapeutic treatment regimens and/or increase of overall survival in subjects treated with other anti-cancer agents, including alkylating agents; antimetabolites; anti-tumor antibiotics; topoisomerase inhibitors; mitotic inhibitors; corticosteroids; targeted therapies; hormone therapy; immunotherapy; and cancer vaccines. Accordingly, the present invention includes the use of GDF15 modulators, such as antibodies to GDF15, in combination with one or more anti-cancer agents, including: abiraterone (*e.g.,* abiraterone acetate); afatinib (*e.g.,* afatinib dimaleate); aflibercept; aldesleukin; alemtuzumab; anastrazole; asparaginase (*e.g*., arasparginase Erwinia chrysanthemi); arsenic (*e.g*., arsenic trioxide); axitinib; azacitidine; belinostat; bendamustine (*e.g.,* bendamustine hydrochloride); bevacizumab (*e.g.,* Avastin®); bicalutamide; bisulfan; bleomycin; bortezomib; bosutinib; brentuximab (*e.g*., brentuximab vedotin); cabazitaxel; cabozantinib (*e.g*., cabozantinib-S-malate); capecitabine; carboplatin; carfilzomib; carmustine; ceritinib; cetuximab; chlorambucil; cisplatin; clofarabine; crizotinib; cyclophosphamide; cytarabine (*e.g*., liposomal cytarabine); dabrafenib; dacarbazine; dactinomycin; dasatinib; daunorubicin (*e.g*., daunorubicin hydrochloride); decitabine; degare; denileukin diftitox; dexamethasone; docetaxel; doxorubicin (*e.g*., Adriamycin®; doxorubicin hydrochloride; doxorubicin hydrochloride liposome); enzalutamide; epirubicin (*e.g*., epirubicin hydrochloride); anti-ErbB2 antibodies; anti-ErbB3 antibodies; erlotinib (*e.g*., erlotinib hydrochloride); etoposide (*e.g*., etoposide phosphate); everolimus; exemestane; anti-FGFR2 antibodies; anti-FGFR3 antibodies; fludarabine (*e.g*., fludarabine phosphate); fluorouracil; fulvestrant; gefitinib; gemcitabine (*e.g.,* gemcitabine hydrochloride); goserelin (*e.g.,* goserelin acetate); anti-HGFl antibodies (*e.g.,* ficlatuzumab); ibrutinib; ibritumomab (*e.g.,* ibritumomab tiuxetan); idelalisib; ifosfamide; imatinib (*e.g*., imatinib mesylate); imiquimod; ipilimumab; irinotecan (*e.g.,* irinotecan hydrochloride); ixabepilone; lapatinib (*e.g.,* lapatinib ditosylate); lenalidomide; letrozole; leucovorin (*e.g.,* leucovorin calcium; folinic acid); leuprolide (*e.g.,* leuprolide acetate); lomustine; mechlorethamine (*e.g*., mechlorethemine hydrochloride); megestrol (*e.g.,* megestrol acetate); mesna; mercaptopurine; methotrexate; mitomycin (*e.g.,* mitomycin C); nelarabine; nilotinib; nivolumab; anti-notchl antibodies; anti-notch3 antibodies; obinutuzumab; ofatumumab; omacetaxine (*e.g*., omacetaxine mepesuccinate); oxaliplatin; paclitaxel (*e.g.,* paclitaxel albumin-stabilized nanoparticle formulation); pamidronate (*e.g.,* pamidronate disodium); panitumumab; pazopanib (*e.g*., pazopanib hydrochloride); pegaspargase; pemetrexed (*e.g*., pemetrexed disodium); pertuzumab; plerixafor; pomalidomide; ponatinib (*e.g.,* ponatanib hydrochloride); pralatrexate; prednisone; procarbazine (*e.g.,* procarbazine hydrochloride); radium 223 (*e.g*., radium 223 dichloride); ramucirumab; recombinant HPV vaccines (*e.g.,* Cervarix®, Gardasil®); recombinant interferon (*e.g.,* interferon alfa-2b; pegylated interferon alfa-2b); pembrolizumab;regorafenib; rituximab; romidepsin; ruxolitinib (*e.g.,* ruxolitinib phosphate); siltuximab; sipuleucel-T; sorafenib (*e.g.,* sorafenib tosylate); sunitinib (*e.g.,* sunitinib malate); tamoxifen (*e.g.,* tamoxifen citrate); temozolomide; temsirolimus; thalidomide; tivozanib; topotecan (*e.g*., topotecan hydrochloride); toremifene; tositumomab (*e.g.,* tositumomab and iodine); trametinib; trastuzumab (*e.g.,* Herceptin®; Kadcyla®); vandetanib; vemurafenib; vinblastine (*e.g*., vinblastine sulfate); vincristine (*e.g*., vincristine sulfate); vismodegib; vorinostat; and zoledronic acid.

The GDF15 modulator may be used with combinations of one or more of the above cancer treatment agents, including but not limited to, the following combinations of anti-cancer agents: AC [Adriamycin (*i.e.,* doxorubicin hydrochloride) + cyclophosphamide]; ACT [Adriamycin® (*i.e.,* doxorubicin hydrochloride) + cyclophosphamide + Taxol® (*i.e.* paclitaxel); CAF [cyclophosphamide + Adriamycin® (*i.e.,* doxorubicin hydrochloride) + fluorouracil]; CMF [cyclophosphamide + methotrexate + fluorouracil]; FEC [fluorouracil + epirubicin hydrochloride + cyclophosphamide]; TAC [Taxotere® (*i.e.* docetaxel) + Adriamycin® (*i.e.,* doxorubicin hydrochloride) + cyclophosphamide]; CAPOX [capecitabine + oxaliplatin]; FOLFIRI [Folinic acid (*i.e.,* leucovorin calcium) + fluorouracil + irinotecan hydrochloride]; FOLFIRI + bevacizumab; FOLFIRI + cetuximab; FOLFOX [Folinic acid (*i.e.,* leucovorin calcium) + fluorouracil + oxaliplatin]; XELOX [Xeloda® (*i.e.,* capecitabine) + oxaliplatin]; Hyper-CVAD [cyclophosphamide + vincristine sulfate + Adriamycin® (*i.e.,* doxorubicin hydrochloride) + dexamethasone]; ADE [Ara-C (*i.e.,* cytarabine) + daunorubicin hydrochloride + etoposide]; chlorambucil + prednisone; CVP [chlorambucil + vincristine sulfate + prednisone]; carboplatin-paclitaxel; carboplatin-taxol; gemcitabine-cisplatin; gemcitabine-oxaliplatin; ABVD [Adriamycin® (*i.e.,* doxorubicin hydrochloride) + bleomycin + vincristine sulfate + dacarbazine]; ABVE [Adriamycin® (*i.e.,* doxorubicin hydrochloride) + bleomycin + vincristine sulfate + etoposide]; ABVE-PC [Adriamycin® (*i.e.,* doxorubicin hydrochloride) + bleomycin + vincristine sulfate + etoposide + prednisone + cyclophosphamide]; BEACOPP [bleomycin + etoposide + Adriamycin® (*i.e.,* doxorubicin hydrochloride) + cyclophosphamide + Oncovin® (*i.e.,* vincristine sulfate + procarbazine hydrochloride + prednisone]; COPP [cyclophosphamide + Oncovin® (*i.e.,* vincristine sulfate + procarbazine hydrochloride + prednisone]; COPP-ABV [cyclophosphamide + Oncovin® (*i.e.,* vincristine sulfate) + procarbazine hydrochloride + prednisone + Adriamycin® (*i.e.,* doxorubicin hydrochloride) + bleomycin +vinblastine sulfate]; ICE [Ifosfamide + carboplatin + etoposide]; MOPP [mechlorethamine hydrochloride + Oncovin® (*i.e.,* vincristine sulfate) + procarbazine hydrochloride + prednisone]; OEPA [Oncovin® (*i.e.,* vincristine sulfate) + etoposide + prednisone + Adriamycin® (*i.e.,* doxorubicin hydrochloride)]; OPPA [Oncovin® (*i.e.,* vincristine sulfate) + procarbazine hydrochloride + prednisone + Adriamycin® (*i.e.,* doxorubicin hydrochloride)]; Stanford V combination [mechloroethamine hydrochloride + doxorubuicin hydrochloride + vinblastine sulfate + vincristine sulfate + bleomycin + etoposide + prednisone]; VAMP [vincristine sulfate + Adriamycin® (*i.e.,* doxorubicin hydrochloride) + methotrexate + prednisone]; CHOP [cyclophosphamide + Hydroxydaunomycin® (*i.e.,* doxorubincin hydrochloride) + Oncovin (*i.e.,* vincristine sulfate) + prednisone]; R-CHOP [rituximab + cyclophosphamide + Hydroxydaunomycin® (*i.e.,* doxorubincin hydrochloride) + Oncovin® (*i.e.,* vincristine sulfate) + prednisone]; EPOCH [etoposide + prednisone + Oncovin® (*i.e.,* vincristine sulfate) + cyclophosphamide + Hydroxydaunomycin® (*i.e.,* doxorubincin hydrochloride)]; PAD [PS-341 (*i.e.,* bortezomib) + Adriamycin® (*i.e.,* doxorubicin hydrochloride) + dexamethasone]; BEP [bleomycin + etoposide + Platinol® (*i.e.,* cisplatin); VeIP [Velban® (*i.e.,* vinblastine sulfate) + ifosfamide + Platinol® (*i.e.,* cisplatin) + mesna]; OFF [oxaliplatin + fluorouracil + Folinic Acid *(i.e.,* leucovorin calcium)].

Exemplary indications for the medical uses of the present disclosure include the following tumors and cancers: breast cancer; lung cancer (including small cell and non-small cell lung cancer); anal, colon, rectal and colorectal cancer; liver cancer; kidney and renal cancer (including renal cell carcinoma); head and neck cancer; pancreatic cancer; bone cancer; cervical, ovarian, vaginal and vulvar cancer; prostate, penile and testicular cancer; anal cancer; bladder cancer; leukemia (including AML; CML; ALL and CLL); stomach cancer (including gastrointestinal stromal tumors) and gastric cancer; brain tumors; gliomas; neuroblastomas and retinoblastomas; thyroid cancer; skin cancer (including melanoma); multiple myeloma (and other plasma cell neoplasms); lymphoma (including Hodgkin's and non-Hodgkin's); sarcoma; myeloproliferative neoplasms; malignant mesothelioma; adult/childhood soft tissue sarcoma; AIDS related Kaposi Sarcoma; endometrial cancer; gestational trophoblastic disease; malignant mesothelioma; multicentric Castleman Disease; myeloproliferative neoplasms; rhabdomyosarcoma; basal cell carcinoma; Wilms tumor and other childhood kidney cancers.

One or more anti-cachexia agents may be used in addition to, or as substitute for, a GDF15 modulator. Anti-cachexia agents that may be useful include megestrol acetate (Agiles et al. (2013) CLINICAL NUTRITION 32:319-324); corticosteroids or glucocorticoids (such as dexamethasone, prednisone, methyl prednisolone); cannabinoids (such as dronabinol); ghrelin and anamorelin; melanocortin antagonists; anti-IL6 monoclonal antibodies; selective androgen receptor modulators (SARS); thalidomide; oxandrolone; activin receptor II; GDF8 (myostatin); and IL-la inhibitors.

### IV: Preferred Embodiments

In preferred embodiments of the invention, subjects may be pre-treated with a GDF15 modulator, such as a GDF15 inhibitory antibody, prior to or concomitant with treatment with one or more anti-cancer agents. Dosage and administration of a GDF15 modulator may be determined by the skilled clinician. In some embodiments, the amount of GDF15 modulator administered to an individual is about 10 µg to about 500 mg per dose, including for example any of about 10 µg to about 50 µg, about 50 µg to about 100 µg, about 100 µg to about 200 µg, about 200 µg to about 300 µg, about 300 µg to about 500 µg, about 500 µg to about 1 mg, about 1 mg to about 10 mg, about 10 mg to about 50 mg, about 50 mg to about 100 mg, about 100 mg to about 200 mg, about 200 mg to about 300 mg, about 300 mg to about 400 mg, or about 400 mg to about 500 mg per dose. In particular preferred embodiments, the GDF15 modulator is a GDF15 antibody selected from the group consisting of Hu01G06-135 and Hu01G06-127. (See WO 2014/100689).

The GDF15 modulator compositions may be administered in a single daily dose, or the total daily dose may be administered in divided dosages of two, three, or four times daily. The compositions can also be administered less frequently than daily, for example, six times a week, five times a week, four times a week, three times a week, twice a week, once a week, once every two weeks, once every three weeks, once a month, once every two months, once every three months, or once every six months. The compositions may also be administered in a sustained release formulation, such as in an implant which gradually releases the composition for use over a period of time, and which allows for the composition to be administered less frequently, such as once a month, once every 2-6 months, once every year, or even a single administration. The sustained release devices (such as pellets, nanoparticles, microparticles, nanospheres, microspheres, and the like) may be administered by injection or surgical implanted in various locations in the body.

In certain preferred embodiments of the invention, the subject is treated with capecitabine (for example, Xeloda®) for cancer of the colon or rectum that has spread to other parts of the body (metastatic colorectal cancer), or cancer of the colon after surgery. Prior to, concomitant with, or subsequent to treatment with capecitabine, the subject is treated with anti-GDF15 antibody as a GDF15 modulator.

In other embodiments, the subject is treated with capecitabine in combination with, or after treatment with, docetaxel (e.g., Taxotere®) for breast cancer that has spread to other parts of the body (metastatic breast cancer). Prior to, concomitant with, or subsequent to treatment with capecitabine, the subject is treated with anti-GDF15 antibody as a GDF15 modulator.

Dosage and administration of capecitabine may be determined by the skilled clinician. A typical regimen may comprise administration of 1250 mg/ml² administered orally twice per day for two weeks, followed by a one week resting period, as a three week cycle. When used in combination with docetaxel, a typical regimen for docetaxel is 75 mg/ml² as one hour intravenous infusion every 3 weeks.

In certain preferred embodiments of the invention, the subject is treated with gemcitabine (for example, Gemzar®), for pancreatic cancer; for ovarian in combination with carboplatin; for breast cancer in combination with paclitaxel; for non-small cell lung cancer (NSCLC) in combination with cisplatin. Prior to, concomitant with, or subsequent to treatment with gemcitabine, the subject is treated with anti-GDF15 antibody as a GDF15 modulator.

Dosage and administration of gemcitabine may be determined by the skilled clinician. A typical regimen may comprise administration of between 1000 and 1250 mg/ml² administered intravenously over 30 minutes on days 1 and 8 of each 21 day cycle; or days 1, 8 and 15 of each 28 day cycle.

In certain preferred embodiments of the invention, the subject is treated with doxorubicin (for example, Adriamycin®) for cancer of the colon or rectum that has spread to other parts of the body (metastatic colorectal cancer), or cancer of the colon after surgery. Prior to, concomitant with, or subsequent to treatment with capecitabine, the subject is treated with anti-GDF15 antibody as a GDF15 modulator.

Doxorubicin (for example Doxil®) is also approved for treatment of ovarian cancer, AIDS-related Kaposi's Sarcoma; and multiple myeloma, in combination with bortezomib, as well as for acute lymphoblastic lymphoma (ALL); acute myeloblastic lymphoma (AML); neuroblastoma; breast carcinoma; ovarian carcinoma; Hodgkin's Disease; malignant lymphoma; and bronchogenic carcinoma in which the small cell type is the most responsive compared to other cell types. Prior to, concomitant with, or subsequent to treatment with doxorubicin, the subject is treated with anti-GDF antibody as a GDF modulator.

Dosage and administration of doxorubicin may be determined by the skilled clinician. A typical regimen may comprise administration of between 50 mg/ml² administered intravenously every 4 weeks, for four courses minimum (ovarian cancer); 20 mg/ml² administered intravenously every three weeks for treatment of AIDS-related Kaposi's Sarcoma. In multiple myeloma, a typical regimen is administration of bortezomib at 1.3 mg/ml², administered as an intravenous bolus injection on days 1, 4, 8 and 11 every 3 weeks, and administration of doxorubicin at 30 mg/ml², administered intravenously on day 4 following the administration of bortezomib.

In certain preferred embodiments of the invention, the subject is treated with carboplatin, for example, Paraplatin®, for ovarian cancer. In other embodiments, the subject is treated with carboplatin in combination with, or after treatment with cyclophosphamide for advanced ovarian cancer. Prior to, concomitant with, or subsequent to treatment with carboplatin, the subject is treated with anti-GDF15 antibody as a GDF15 modulator.

Dosage and administration of carboplatin may be determined by the skilled clinician. A typical regimen may comprise administration 300-360 mg/ml² intravenous on day 1 every 4 weeks for approximately 6 cycles. When cyclophosphamide is co-administered, a typical regimen may be 300 mg/ml² intravenous infusion of carboplatin one day every 4 weeks for 6 cycles, combined with 600 mg/ml² intravenous infusion of cyclophosphamide one day every 4 weeks for 6 cycles.

In certain preferred embodiments of the invention, the subject is treated with cisplatin, for example, Platinol®, for the treatment of metastatic testicular tumors, metastatic ovarian tumors, or advanced bladder cancer. In other embodiments, the subject is treated with cisplatin in combination with, or after treatment with cyclophosphamide. Prior to, concomitant with, or subsequent to treatment with cisplatin, the subject is treated with anti-GDF15 antibody as a GDF15 modulator.

Dosage and administration of cisplatin may be determined by the skilled clinician. A typical regimen may comprise administration 20 mg/ml² intravenous daily for 5 days per cycle for metastatic testicular tumors. For advanced bladder cancer, a typical regimen for cisplatin may comprise 50-70 mg/ml² intravenous infusion once every 3 to 4 weeks, depending upon the extent of prior exposure to radiation therapy and/or prior chemotherapy. For heavily pretreated patients, a dose of 50 mg/ml² intravenous once every 4 weeks is typical. For treatment of metastatic ovarian tumors, 75 to 100 mg/ml² intravenous per cycle once every 4 weeks is typical. When cisplatin administration is combined with cyclophosphamide, cisplatin injection and cyclophosphamide should be administered sequentially. A typical regimen may be 600 mg/ml² intravenous infusion of cyclophosphamide on day 1 every 4 weeks.

In certain preferred embodiments of the invention, the subject is treated with oxaliplatin, for example Eloxatin®, in combination with 5-fluorouracil and/or leucovorin, for treatment of cancer of the colon or advanced colorectal cancer, or cancer of the colon after surgery. Prior to, concomitant with, or subsequent to treatment with oxaliplatin, the subject is treated with anti-GDF15 antibody as a GDF15 modulator.

Dosage and administration of oxaliplatin may be determined by the skilled clinician. A typical regimen may comprise administration 85 mg/ml² intravenous infusion of oxaliplatin in 250-500 ml 5% dextrose, over 120 minutes, at the same time as 200 mg/ml² intravenous infusion of leucovorin, followed by 400 mg/ml² of 5-fluorouracil intravenous bolus given over 4-6 minutes.

### EXAMPLES

The following Examples are merely illustrative and are not intended to limit the scope or content of the invention in any way.

### Example 1: Inhibition of GDF15 in Cancer Cachexia Tumor-Bearing Mice:

This Example demonstrates the increase in overall survival of mice bearing LNCaP prostate xenograft model when treated with a GDF15 modulator in combination with an anti-cancer agent (*e.g*., tivozanib). LNCaP cells were grown in culture at 37°C in an atmosphere containing 5% CO₂, using RPMI-1640 Medium (ATCC® 30-2001™) containing 10% FBS. Cells were inoculated subcutaneously into the flank of 8-week old female NCR Nude mice with 5 x 106 cells per mouse in 50% matrigel. When tumor size reached 500 mm³, the mice were randomized into three groups of ten mice each. Each group received one of the following treatments: (1) murine immunoglobulin G (20 mpk) and vehicle (Control); (2) murine anti-GDF15 antibody 14F11 (20 mpk) and tivozanib (5 mpk); or (3) murine immunoglobulin G (20 mpk) and tivozanib (5 mpk). Antibodies were administered every 3 days by intra-peritoneal injection, tivozanib and vehicle control was administered daily by oral gavage. Body weight and tumor size were measured daily. Mice were evaluated daily and if any of the following criteria were achieved, animals were sacrificed: a) tumor size larger than 2,000 mm³; b) body weight loss greater than 20%; c) moribund.

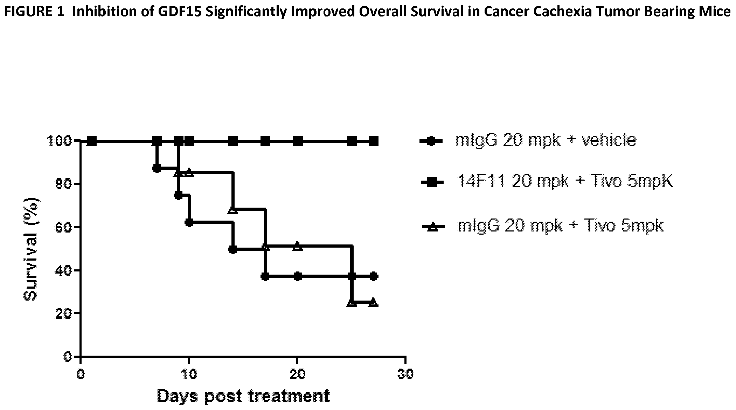

Treatment with the anti-tumor agent tivozanib, an angiogenesis inhibitor, slows down tumor growth. In the absence of GDF15 inhibition, 70% of the mice die due to cachexia over a period of 30 days, even in the presence of anti-cancer treatment. However, as shown in FIG. 1, the combination of GDF15 inhibition with anti-cancer agent tivozanib reverses cachexia, and results in 100% sustained survival, and effective anti-tumor treatment over the 30 day period.

### Example 2: Inhibition of GDF15 in Cisplatin-Induced Cachexia Model:

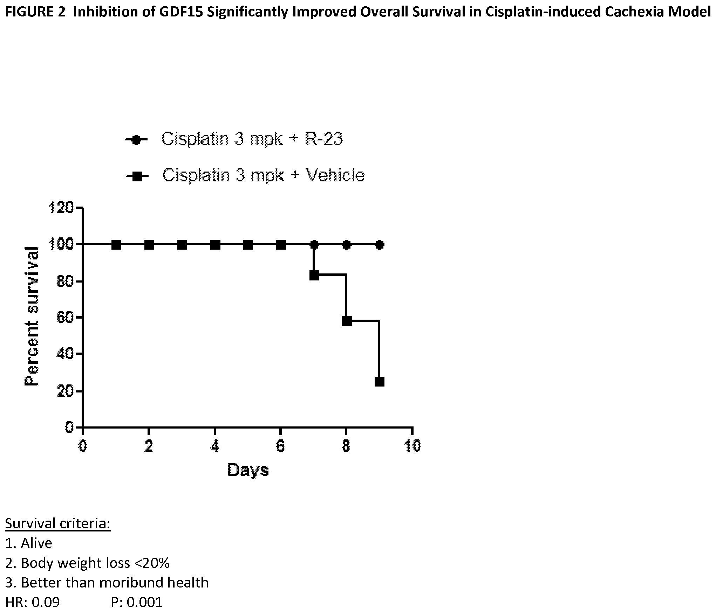

This Example demonstrates the increase in overall survival of mice treated with an anti-cancer agent (*e.g*., cisplatin) when a GDF15 modulator is administered. Naive non-tumor-bearing, 8-week old, female, ICR-Scid mice were treated with cisplatin (3 mpk) twice a week by intra-peritoneal injection. Body weight was measured daily. After 2 doses of cisplatin (day 0 of the experiment) mice were randomized into two groups of ten mice each. One group received cisplatin 3 mpk plus (1) vehicle; or cisplatin 3 mpk with (2) rabbit monoclonal antibody raised against murine anti-GDF15 antibody, R-23 twice a week (20 mpk) by intra-peritoneal injection. In the absence of GDF15 inhibition, 80% of the mice die due to cachexia caused by the cisplatin agent over a period of 9 days. However, as shown in FIG. 2, the combination of GDF15 inhibition with cisplatin treatment resulted in 100% sustained survival over the 9 day period.

### Example 3: Inhibition of GDF15 in Carboplatin-Induced Cachexia Model:

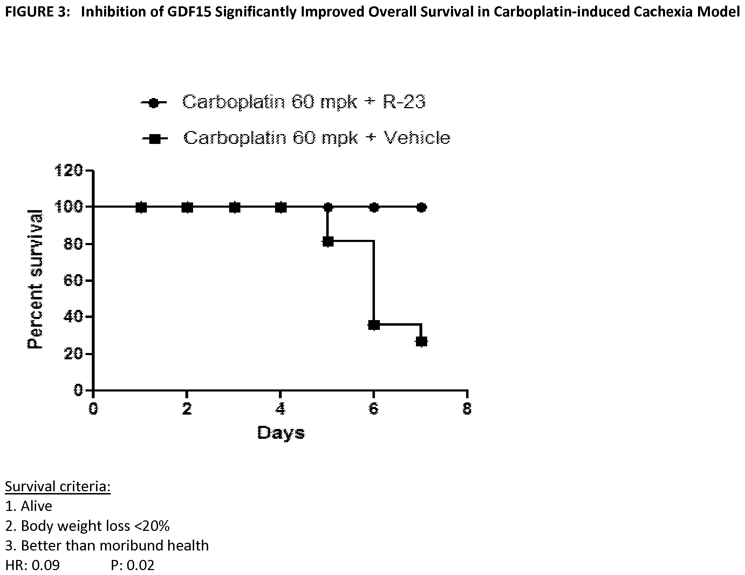

This Example demonstrates the increase in overall survival of mice treated with an anti-cancer agent (*e.g*., carboplatin) when a GDF15 modulator is administered. Naive non-tumor-bearing, 8-week old, female, ICR-Scid mice were treated with carboplatin (60 mpk) by intra-peritoneal injection on Day 0 and Day 3 of this experiment. On Day 2, after mice experience 8 % body weight loss, mice were randomized into two groups of ten mice each. One group received carboplatin (60 mpk) plus (1) vehicle or carboplatin (60 mpk) plus (2) rabbit monoclonal antibody raised against murine anti-GDF15 antibody, R-23 (20 mpk) by intra-peritoneal injection on Day 2 and Day 4. In the absence of GDF15 inhibition, 80% of the mice died due to cachexia caused by the carboplatin agent over a period of 8 days. However, as shown in FIG. 3, the combination of GDF15 inhibition with carboplatin treatment resulted in sustained survival over the 8 day period.

### Example 4: Inhibition of GDF15 in Oxaliplatin-Induced Cachexia Model:

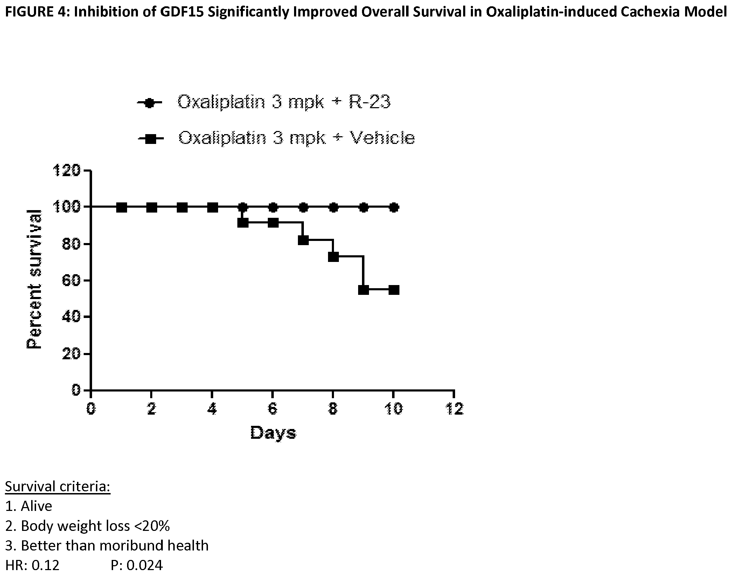

This Example demonstrates the increase in overall survival of mice treated with an anti-cancer agent (*e.g*., oxaliplatin) when a GDF15 modulator is administered. Naive non-tumor-bearing, 8-week old, female, ICR-Scid mice were treated daily with oxaliplatin (3 mpk) by intra-peritoneal injection. On Day 3, after mice experience 8 % body weight loss, mice were randomized into two groups of ten mice each. One group received oxaliplatin (3 mpk) plus (1) vehicle; or oxaliplatin (3 mpk) plus (2) rabbit monoclonal antibody raised against murine anti-GDF15 antibody, R-23 (20 mpk). Antibody or vehicle was dosed on Day 3, 5, 7, 9 by intra-peritoneal injection. In the absence of GDF15 inhibition, 45% of the mice die due to cachexia caused by the oxaliplatin agent over a period of 10 days. However, as shown in FIG. 4, the combination of GDF15 inhibition with oxaliplatin treatment resulted in sustained survival over the 10 day period.

## Claims

1. At least one chemotherapeutic anti-cancer agent and at least one anti-GDF15 antibody or GDF15-binding fragment thereof for use in treating chemotherapy induced cachexia by increasing the overall survival in a subject having chemotherapy-induced cachexia.

2. The at least one anti-cancer agent and the at least one anti-GDF15 antibody or GDF15-binding fragment thereof for the use of claim 1, wherein the anti-cancer agent is selected from the group consisting of: capecitabine, gemcitabine, doxorubicin, cisplatin, carboplatin and oxaliplatin.

3. The at least one chemotherapeutic anti-cancer agent and the at least one anti-GDF15 antibody or GDF15-binding fragment thereof for the use of claim 1, wherein administration of the anti-GDF15 antibody or GDF15-binding fragment thereof and the chemotherapeutic anti-cancer agent prolongs mean survival in a first patient population with chemotherapy-induced cachexia relative to a second patient population with chemotherapy-induced cachexia who do not receive the GDF15 modulator.

## Patentansprüche

1. Mindestens ein chemotherapeutisches Antikrebsmittel und mindestens ein anti-GDF15-Antikörper oder GDF15-bindendes Fragment davon zur Verwendung bei der Behandlung von chemotherapieinduzierter Kachexie durch Erhöhen des Gesamtüberlebens bei einem Individuum mit chemotherapieinduzierter Kachexie.

2. Das mindestens eine Antikrebsmittel und der mindestens eine anti-GDF15-Antikörper oder das GDF15-bindende Fragment davon zur Verwendung nach Anspruch 1, wobei das Antikrebsmittel ausgewählt ist aus der Gruppe bestehend aus: Capecitabin, Gemcitabin, Doxorubicin, Cisplatin, Carboplatin und Oxaliplatin.

3. Das mindestens eine chemotherapeutische Antikrebsmittel und der mindestens eine anti-GDF15-Antikörper oder das GDF15-bindende Fragment davon zur Verwendung nach Anspruch 1, wobei die Verabreichung des anti-GDF15-Antikörpers oder des GDF15-bindenden Fragments davon und des chemotherapeutischen Antikrebsmittels das mittlere Überleben in einer ersten Patientenpopulation mit chemotherapieinduzierter Kachexie im Vergleich zu einer zweiten Patientenpopulation mit chemotherapieinduzierter Kachexie, die den GDF15-Modulator nicht erhält, verlängert.

## Revendications

1. Au moins un agent anticancéreux chimiothérapeutique et au moins un anticorps anti-GDF15 ou fragment de liaison de GDF15 de celui-ci pour l'utilisation dans le traitement d'une cachexie induite par chimiothérapie en augmentant la survie globale chez un sujet ayant une cachexie induite par chimiothérapie.

2. Le au moins un agent anticancéreux et le au moins un anticorps anti-GDF15 ou fragment de liaison de GDF15 de celui-ci pour l'utilisation selon la revendication 1, dans lequel l'agent anticancéreux est choisi dans le groupe constitué de : capécitabine, gemcitabine, doxorubicine, cisplatine, carboplatine et oxaliplatine.

3. Le au moins un agent anticancéreux chimiothérapeutique et le au moins un anticorps anti-GDF15 ou fragment de liaison de GDF15 de celui-ci pour l'utilisation selon la revendication 1, dans lequel une administration de l'anticorps anti-GDF15 ou du fragment de liaison de GDF15 de celui-ci et de l'agent anticancéreux chimiothérapeutique prolonge la survie moyenne dans une première population de patients avec une cachexie induite par chimiothérapie par rapport à une deuxième population de patients avec une cachexie induite par chimiothérapie qui ne reçoivent pas le modulateur de GDF15.
